# EUROPÄISCHE PATENTANMELDUNG

(11) **EP 4 502 768 A1**
(43) Veröffentlichungstag der Anmeldung: **05.02.2025**
(21) Anmeldenummer: 24189097.9
(22) Anmeldetag: 17.07.2024
(51) Int. Cl.: G06F 3/01, A61B 5/00, G16H 20/70

(54) **SYSTEM ZUR DYNAMISCHEN STEUERUNG EINER AUSGABEUMGEBUNG**

(30) Priorität: 31.07.2023 AT 506122023
(71) Anmelder: Ars Electronica Linz GmbH & Co KG, 4040 Linz (AT)
(72) Erfinder: Kiesenhofer, Susanne, 4040 Linz (AT); Oelsch, Anna, 4040 Linz (AT); Rammer, Daniel, 4040 Linz (AT)
(74) Vertreter: Schwarz & Partner Patentanwälte GmbH

(57) **Zusammenfassung**

System (1) zur dynamischen Steuerung einer Ausgabeumgebung (2) umfassend eine Rechnereinheit (3), und zumindest ein durch die Rechnereinheit (3) steuerbares Ausgabemittel (4), wobei das System (1) dazu ausgebildet ist mittels des Ausgabemittels (4) in der Ausgabeumgebung (2) eine durch die Rechnereinheit (3) generierte Ausgabe auszugeben, wobei das System (1) zumindest zwei, an jeweils einer Person anbringbare Wearables (5) umfasst, wobei die Wearables (5) mit der Rechnereinheit (3) verbunden, und dazu ausgebildet sind, zumindest einen physiologischen Parameter der das jeweilige Wearable (5) tragenden Person im Wesentlichen kontinuierlich zu erfassen und an die Rechnereinheit (3) zu übertragen, und die Rechnereinheit (3) dazu ausgebildet ist, die Ausgabe anhand aller durch die Wearables (5) erfassten physiologischen Parameter zu steuern.

## Beschreibung

Die Erfindung betrifft ein System zur dynamischen Steuerung einer Ausgabeumgebung umfassend eine Rechnereinheit, und zumindest ein durch die Rechnereinheit steuerbares Ausgabemittel, wobei das System dazu ausgebildet ist mittels des Ausgabemittels in der Ausgabeumgebung eine durch die Rechnereinheit generierte Ausgabe auszugeben.

Systeme zur Steuerung einer Ausgabeumgebung sind am Markt in einer Vielzahl unterschiedlicher Ausführungsvarianten verfügbar. Im Stand der Technik bekannte Systeme umfassen eine Rechnereinheit und zumindest ein durch die Rechnereinheit steuerbares Ausgabemittel. Dieses Ausgabemittel kann beispielsweise als Virtual- oder Augmented-Reality Brille ausgeführt sein. Es sind auch Systeme bekannt, welche als Ausgabemittel große Bildschirme oder Projektoren aufweisen, welche in begehbaren Räumen eingesetzt werden, um eine künstliche Umgebung zu simulieren. Das Ausgabemittel ist somit dazu ausgebildet in der Ausgabeumgebung eine durch die Rechnereinheit generierte Ausgabe auszugeben, und somit die virtuelle Umgebung zu generieren. Derartige Systeme sind am Markt unter den Schlagworten Virtual Reality-, Augmented Reality-, Mixed Reality- und/oder Extended Reality Systeme bekannt.

Derartige Systeme können auch interaktiv, beispielsweise mittels einer Benutzer*inneneingabe gesteuert werden. Die Benutzer*inneneingabe erfolgt herkömmlicherweise entweder über einen Controller oder eine Gestensteuerung, wodurch von einem Benutzer Befehle an die Rechnereinheit generiert werden können.

Ein Nachteil derartiger Systeme liegt jedoch darin, dass diese nur über eingeschränkte Feedbackmechanismen zwischen dem/der Benutzerin und den dargestellten Inhalten verfügen. Der/Die Benutzerin muss in der Regel eine aktive Eingabe tätigen, um Befehle an die Rechnereinheit zu geben. Des Weiteren ermöglichen Systeme gemäß dem Stand der Technik nur in einem sehr eingeschränkten Umfang die Einbindung von Benutzergruppen, welche herkömmlicherweise nur in der Gruppengesamtheit, und nicht als Individuen wahrgenommen werden.

Der Erfindung liegt die Aufgabe zugrunde, ein System zur dynamischen Steuerung einer Ausgabeumgebung bereitzustellen, welches diese Nachteile des Standes der Technik vermeidet.

Erfindungsgemäß wird diese Aufgabe dadurch gelöst, dass das System zumindest zwei, an jeweils einer Person anbringbare Wearables umfasst, wobei die Wearables mit der Rechnereinheit verbunden, und dazu ausgebildet sind, zumindest einen physiologischen Parameter der das jeweilige Wearable tragenden Person im Wesentlichen kontinuierlich zu erfassen und an die Rechnereinheit zu übertragen. Die Rechnereinheit ist zudem dazu ausgebildet, die Ausgabe anhand aller durch die Wearables erfassten physiologischen Parameter zu steuern.

Hierdurch wird der Vorteil erreicht, dass jede Person einer Benutzer*innengruppe in das erfindungsgemäße System eingebunden wird, und der zumindest eine physiologische Parameter jeder der Personen automatisch und im Wesentlichen kontinuierlich erfasst wird. Hierdurch wird eine unbewusste und automatisierte Steuerung der Ausgabeumgebung durch alle Nutzer*innen gemeinsam erreicht, wodurch ein kontinuierlicher Input-Output Loop generiert werden kann. Im Wesentlichen kontinuierlich ist im Rahmen der Erfindung als eine Erfassung des physiologischen Parameters mit einem statischen oder auch einem dynamischen Samplingintervall zu verstehen.

Vorzugsweise weist das erfindungsgemäße System im Bereich der Ausgabeumgebung angeordnete, mit der Rechnereinheit verbundene Erfassungsmittel zur Erfassung von Umweltbedingungen in der Ausgabeumgebung und/oder physiologischen Parametern von in der Ausgabeumgebung befindlichen Personen auf, wobei die Rechnereinheit dazu ausgebildet ist, die Ausgabe anhand aller durch die Wearables und die Erfassungsmittel erfassten physiologischen Parameter und durch die Erfassungsmittel erfassten Umweltbedingungen zu steuern. Hierdurch wird der Vorteil erreicht, dass auch die Umweltbedingungen in der Ausgabeumgebung Einfluss auf die Ausgabe haben.

Gemäß der bevorzugten Ausführungsvariante des erfindungsgemäßen Systems umfasst der von dem jeweiligen Wearable erfasste physiologische Parameter der Person Puls, Körpertemperatur, elektrodermale Aktivität, Sauerstoffsättigung im Blut, EEG-Daten, EKG-Daten, Audiodaten, Beschleunigungsmessdaten, Lagedaten, Magnetdaten Positionsdaten, Augenposition, Blickrichtung, und/oder Atemfrequenz der Person. Hierdurch wird der Vorteil erreicht, dass ein großer Umfang an Parametern der Benutzer*innengruppe, die unter anderem auf unbewusste und unterbewusste, biologische und physiologische Prozesse rückschließen lassen, zur Steuerung des erfindungsgemäßen Systems bereitgestellt wird.

Vorzugsweise ist die Rechnereinheit zusätzlich dazu ausgebildet, aus allen von den Wearables erfassten physiologischen Parametern aller jeweils mindestens ein Wearable tragenden Personen ein statistisches Profil zu erstellen, und die Ausgabe anhand des statistischen Profils zu steuern. Hierdurch wird ermöglicht, dass das erfindungsgemäße System nicht nur anhand der individuellen physiologischen Parameter der einzelnen Personen gesteuert wird, sondern auch gruppendynamische Parameter der Nutzer*innengruppe erfasst und verwendet werden können, um das System zu steuern. Hierdurch kann das System automatisch auf eine Reaktion der gesamten Benutzer*innengruppe reagieren. Vorzugsweise umfasst die Erstellung des statistischen Profils die Erfassung eines Mittelwerts, eines gewichteten Mittelwerts, einer Schiefe und/oder einer Standardabweichung der erfassten physiologischen Parameter der Personen.

Vorteilhaft umfassen die Wearables jeweils zumindest ein mit der Rechnereinheit verbundenes Ausgabemittel, welches dazu ausgebildet ist, eine von der Rechnereinheit generierte Ausgabe mittels des Ausgabemittels des Wearables direkt an die das Wearable tragende Person auszugeben. Hierdurch kann ein direktes Feedback an eine spezifische Person übermittelt werden. Dieses Ausgabemittel der Wearables kann ein Anzeigemittel, einen Lautsprecher und/oder ein haptisches Ausgabegerät wie eine Vibrationseinheit umfassen. Des Weiteren kann das Anzeigemittel beispielsweise ein Heizelement oder einen Ventilator umfassen.

Gemäß der bevorzugten Ausführungsvariante des erfindungsgemäßen Systems umfasst jedes Wearable ein Armband, einen Brustgurt, eine Brille, ein Stirnband, einen Hut, eine Kopfbedeckung, ein Halsband und/oder eine Halskette. Hierdurch wird eine Vielzahl unterschiedlicher Befestigungsmöglichkeiten für das Wearable an der jeweiligen Person bereitgestellt.

Vorzugsweise umfasst das erfindungsgemäße System zudem mit der Rechnereinheit verbundene Positionserfassungssensoren, welche dazu ausgebildet sind, eine Position der Personen in der Ausgabeumgebung zu erfassen. Die Rechnereinheit ist überdies vorzugsweise dazu ausgebildet, die Ausgabe anhand der Position der zumindest einen Person zu steuern. Hierdurch wird der Vorteil erreicht, dass aus der Position der Personen in der Ausgabeumgebung mittels der Rechnereinheit das Ausgabemittel gesteuert werden kann. Vorzugsweise umfassen die Positionserfassungssensoren LiDAR-Sensoren, Kameras, Netzwerktriangulationsvorrichtungen, Bluetoothtriangulationsvorrichtungen, und/oder UWB Triangulationsvorrichtungen. Des Weiteren können die Wearables auch inertiale Messeinheiten umfassen, welche mit einem oder mehreren der vorgenannten Positionserfassungssensoren kombiniert werden können. Hierdurch wird der Vorteil erreicht, dass eine Pose bzw. eine Körperhaltung oder ein Bewegungsablauf einer Person mit der absoluten oder relativen Position der Person im Raum verknüpft werden kann.

Die Wearables sind mit der Rechnereinheit vorzugsweise per WLAN, Bluetooth, Kabel, Mobilfunknetz und/oder per direkter Funkverbindung verbunden. Hierdurch steht eine Vielzahl an Verbindungsmöglichkeiten bereit.

Die Wearables können zudem jeweils mindestens ein Eingabemittel zur manuellen Eingabe eines Steuerungsbefehls und zur Übermittlung des Steuerungsbefehls an die Rechnereinheit umfassen. Hierdurch wird eine aktive Eingabemöglichkeit an die einzelnen Benutzer zur Verfügung gestellt.

Vorteilhafte Ausgestaltungen des erfindungsgemäßen Systems und alternative Ausführungsvarianten werden in weiterer Folge anhand der Figuren im Detail erläutert.

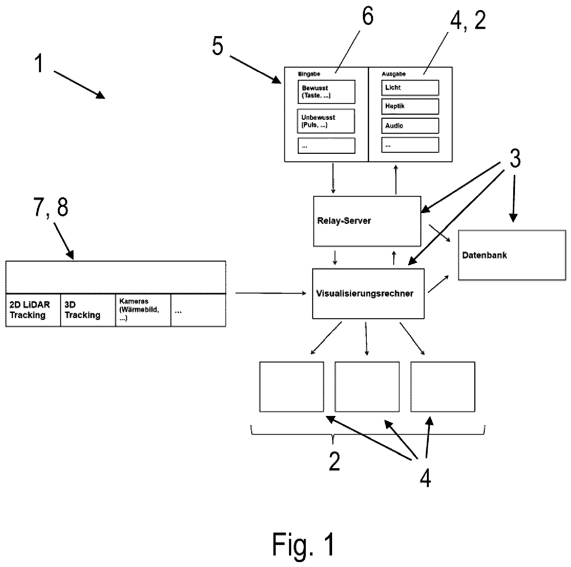

Figur 1 zeigt eine Prinzipskizze einer bevorzugten Ausführungsvariante eines erfindungsgemäßen Systems zur dynamischen Steuerung einer Ausgabeumgebung.

Das in Figur 1 in einer bevorzugten Ausführungsvariante dargestellte erfindungsgemäße System 1 zur dynamischen Steuerung einer Ausgabeumgebung 2 umfasst eine Rechnereinheit 3, und zumindest ein durch die Rechnereinheit steuerbares Ausgabemittel 4. Die Rechnereinheit 3 kann beispielsweise in Form eines Servers bereitgestellt sein, welcher mit dem Ausgabemittel 4 verbunden ist. Das Ausgabemittel 4 kann beispielsweise eine Projektionseinheit, ein Bildschirm oder auch eine Virtual-Reality Brille oder eine Augmented-Reality Brille sein. Naturgemäß kann das erfindungsgemäße System 1, wie in Figur 1 dargestellt, auch mehrere Ausgabemittel 4 in der Ausgabeumgebung 2 umfassen. Gemäß einer bevorzugten Ausführungsform der Erfindung werden als Ausgabemittel 4 Projektionseinheiten, Bildschirme und Lautsprecher verwendet. Das erfindungsgemäße System 1 ist dazu ausgebildet mittels des Ausgabemittels 4 in der Ausgabeumgebung 2 eine durch die Rechnereinheit 3 generierte Ausgabe auszugeben. Die Ausgabeumgebung 2 kann hierbei ein realer Raum sein, in welchem die Ausgabemittel 4 installiert sind. Alternativ kann die Ausgabeumgebung 2 auch vollständig oder teilweise virtuell sein und durch eine oder mehrere Ausgabemittel 4 wie Virtual-Reality Brillen oder einen oder mehrere Bildschirme oder Projektoren dargestellt werden. Auch Augmented-Reality, Mixed Reality- und/oder Extended Reality Brillen sind als Ausgabemittel 4 im Rahmen der Erfindung einsetzbar. Gemäß einer Ausführungsvariante kann die Ausgabeumgebung 2 somit ein Raum sein, in welchem die Ausgabemittel 4 installiert sind, oder in welchen die Ausgabemittel 4 durch die Benutzerinnen eingebracht werden. Hierdurch kann eine Vielzahl verschiedener Ausgabemittel 4 genutzt werden, um das Erlebnis eines Benutzers oder einer Benutzerin des erfindungsgemäßen Systems 1 zu gestalten. Das System 1 umfasst erfindungsgemäß zumindest zwei, an jeweils einer Person anbringbare Wearables 5, wobei die Wearables 5 mit der Rechnereinheit 3 verbunden, und dazu ausgebildet sind, zumindest einen physiologischen Parameter der das jeweilige Wearable 5 tragenden Person im Wesentlichen kontinuierlich zu erfassen und an die Rechnereinheit 3 zu übertragen. Das Ausgabemittel 4 kann auch an den Wearables 5 vorgesehen sein. Die Rechnereinheit 3 ist vorzugsweise als Server ausgeführt. Die Rechnereinheit 3 ist dazu ausgebildet ist, die Ausgabe anhand aller durch die Wearables 5 erfassten physiologischen Parameter zu steuern. Die erfassten Parameter können hierbei unter anderem Puls, Körpertemperatur, elektrodermale Aktivität, Sauerstoffsättigung im Blut, EEG-Daten, EKG-Daten, Audiodaten, Beschleunigungsmessdaten, Lagedaten, Positionsdaten, Magnetdaten, Augenposition, Blickrichtung, und/oder Atemfrequenz umfassen. Hierzu weisen die Wearables 5 entsprechende Sensoren wie beispielsweise inertiale Messeinheiten und/oder Biosensoren auf, welche diese Parameter erfassen. Des Weiteren kann das Wearable 5 beispielsweise als Brille ausgebildet sein, und einen in Figur 1 nicht gesondert ersichtlichen Augenbewegungssensor umfassen. Hierdurch wird es ermöglicht festzustellen, welche Person, welchen Bereich der in der Ausgabeumgebung 2 generierten Ausgabe wie viel Aufmerksamkeit schenkt. Aus den erfassten Parametern kann beispielsweise auch eine emotionale Reaktion, der das jeweilige Wearable 5 tragende Person erkannt werden. Als im Wesentlichen kontinuierliche Erfassung wird im Rahmen der Erfindung eine der Fachperson allgemein bekannte Erfassung der genannten Parameter mit einem für derartige Sensoren üblichen Samplingintervall verstanden.

Hierdurch wird der Vorteil erreicht, dass die Ausgabe, und somit die in der Ausgabeumgebung 2 dargestellten Inhalte anhand von Daten jeder einzelnen Person gesteuert werden können, welche das erfindungsgemäße System 1 nutzen. Hierdurch wird ermöglicht einen Feedbackloop zwischen der Ausgabe in der Ausgabeumgebung 2 und der Reaktion der Benutzerinnen zu generieren, wobei entweder auf Reaktionen der gesamten Benutzer*innengruppe oder auch nur auf die Reaktion eines Mitglieds der Benutzer*innengruppe eingegangen werden kann.

Die Wearables 5 können jeweils beispielsweise ein Armband, einen Brustgurt, eine Brille, ein Stirnband, eine Kopfbedeckung, einen Hut, ein Halsband und/oder eine Halskette umfassen. Zudem ist es auch möglich, dass ein Wearable 5 mehrere dieser Komponenten umfasst. Vorzugsweise sind die Wearables 5 mit der Rechnereinheit 3 per WLAN, Bluetooth, Kabel, per Mobilfunknetz und/oder direkte Funkverbindung verbunden. Weitere alternative Verbindungsmethoden sind der Fachperson allgemein bekannt. Die Wearables 5 können zudem jeweils zumindest ein Eingabemittel 6 zur manuellen Eingabe eines Steuerungsbefehls und zur Übermittlung des Steuerungsbefehls an die Rechnereinheit 3 umfassen. Diese Eingabemittel 6 kann beispielsweise in Form eines oder mehrerer Touchscreens oder eines oder mehrere Druckknöpfe bereitgestellt sein. Das Eingabemittel 6 kann auch in Form eines oder mehrerer Mikrophone mit einer Software zur Spracherkennung und Sprachsteuerung bereitgestellt sein. Hierdurch wird es den Personen ermöglicht aktive Eingaben an das System zu tätigen. Gemäß einer Ausführungsvariante des erfindungsgemäßen Systems 1 können zusätzlich zu den Wearables 5 auch handheld-Geräte wie beispielsweise Gamecontroller vorgesehen sein, welche mit der Rechnereinheit 3 verbunden sind, wobei die handheld-Geräte alternativ oder zusätzlich zu den Wearables 5 ebenfalls derartige Eingabemittel 6 aufweisen können.

Vorzugsweise ist die Rechnereinheit 3 dazu ausgebildet, aus allen von den Wearables 5 erfassten physiologischen Parametern aller, jeweils mindestens ein Wearable 5 tragenden Personen ein statistisches Profil zu erstellen, und die Ausgabe anhand des statistischen Profils zu steuern. Zur Erstellung des statistischen Profils kann jegliche mathematische Methodik aus der Statistik angewandt werden, um Daten von Interesse zu generieren. Hierdurch können gruppendynamische Parameter der Nutzer*innengruppe erfasst und verwendet werden, um das erfindungsgemäße System 1 zu steuern. Vorzugsweise umfasst die Erstellung des statistischen Profils beispielsweise die Erfassung eines Mittelwerts, eines gewichteten Mittelwerts, der Schiefe, und/oder einer Standardabweichung der erfassten physiologischen Parameter der Personen. Hierdurch eignet sich das erfindungsgemäße System 1 auch zum Einsatz beispielsweise in soziologischen Studien beziehungsweise zur Analyse von gruppendynamischen Prozessen.

Die Wearables 5 selbst können darüber hinaus ebenfalls jeweils zumindest ein mit der Rechnereinheit 3 verbundenes Ausgabemittel 4 umfassen. Diese kann genutzt werden, um eine von der Rechnereinheit 3 generierte Ausgabe mittels des Ausgabemittels 4 des Wearables 5 direkt an die das Wearable 5 tragende Person auszugeben. Dieses Ausgabemittel 4 des Wearables 5 umfasst vorzugsweise ein Anzeigemittel, einen Lautsprecher und/oder ein haptisches Ausgabegerät wie eine Vibrationseinheit umfassen. Des Weiteren kann das Anzeigemittel beispielsweise ein Heizelement oder einen Ventilator umfassen. Hierdurch können Ausgaben direkt an eine einzige Person einer Gruppe an Benutzerinnen des erfindungsgemäßen Systems 1 ausgegeben werden.

Zur weiteren Verbesserung der Ausgabe des erfindungsgemäßen Systems 1 kann das System 1 vorzugsweise Positionserfassungssensoren 7 umfassen. Diese Positionserfassungssensoren 7 erfassen eine Position der Personen in der Ausgabeumgebung 2. Die Positionserfassungssensoren 7 können hierzu beispielsweise LiDAR Sensoren oder Kameras wie RGB-, Graustufen- und/oder Tiefenkameras umfassen. Auch 3D point-cloud-trackingmittel sind im Rahmen des erfindungsgemäßen Systems 1 als Positionserfassungssensoren 7 implementierbar. Die Positionserfassungssensoren 7 sind ebenfalls mit der Rechnereinheit 3 verbunden, und die Rechnereinheit 3 steuert vorzugsweise die Ausgabe anhand von den Positionserfassungssensoren 7 erfassten Positionen aller Personen in der Ausgabeumgebung 2. Alternativ kann auch markerbasiertes Tracking eingesetzt werden, in welchem die Personen mit aktiven oder passiven Markern versehen werden, welche mittels Kameras erfasst werden. In Kombination mit einer 3D Brille als Wearable 5 und einem Eye-Tracking System kann folglich die genaue Blickrichtung jeder Person errechnet werden. Die Position der Personen kann beispielsweise mittels Bildanalyse in den Bildern der Kameras erkannt werden. Hierdurch kann beispielsweise auch die Körperhaltung einzelner oder jeder Person erfasst werden. Alternative Positionserfassungssensoren 7 sind der Fachperson allgemein bekannt.

Durch die Erkennung der Position j eder Person in der Ausgabeumgebung 2 kann die Ausgabe durch die Rechnereinheit 3 an diese Position angepasst werden.

Vorzugsweise weist das erfindungsgemäße System 1 zudem im Bereich der Ausgabeumgebung 2 angeordnete, mit der Rechnereinheit 3 verbundene Erfassungsmittel 8 zur Erfassung von Umweltbedingungen in der Ausgabeumgebung 2 und/oder physiologischen Parametern von in der Ausgabeumgebung 2 befindlichen Personen auf. Diese Erfassungsmittel 8 können beispielsweise durch Wärmebildkameras, Thermometer, Hygrometer, etc. ausgebildet sein. Die Rechnereinheit 3 ist hierbei dazu ausgebildet ist, die Ausgabe anhand aller durch die Wearables 5 und die Erfassungsmittel 8 erfassten physiologischen Parameter und durch die Erfassungsmittel 8 erfassten Umweltbedingungen zu steuern. Hierdurch wird der Vorteil erreicht, dass auch die Umweltbedingungen in der Ausgabeumgebung 2 Einfluss auf die Ausgabe haben. Zudem ist es hierdurch beispielsweise möglich mittels eines Feedbackloops die Position und Parameter der einzelnen Personen in einer Gruppe zu beeinflussen, oder anhand der Positionierung und der physiologischen Parameter der einzelnen Personen eine Relation der Personen innerhalb der Gruppe abzuleiten. Das erfindungsgemäße System 1 kann des Weiteren dazu ausgebildet sein, die erfassten Parameter der Personen zu speichern. Dies kann beispielsweise mittels einer Datenbank realisiert werden, die Teil der Rechnereinheit 3 ist oder mit der Rechnereinheit 3 verbunden ist. Hierdurch stehen diese für eine spätere Analyse zur Verfügung. Gemäß einer Ausführungsvariante des erfindungsgemäßen Systems 1 werden die erfassten Parameter von der Rechnereinheit 3 an die Wearables 5 der Personen übermittelt und an diesen angezeigt. Hierdurch können die Personen die Parameter einsehen, wodurch diese beispielsweise auf spezifische Zustände einer oder mehrerer anderer Personen innerhalb der Benutzer*innengruppe reagieren können. Durch die vielfältigen Feedbackmöglichkeiten des erfindungsgemäßen Systems 1 eignet sich dieses beispielsweise als Mediationstool und auch als Trainingstool für die Behandlung von Angstzuständen. Des Weiteren kann durch das erfindungsgemäße System 1 das Zusammengehörigkeitsgefühl innerhalb einer Benutzer*innengruppe gestärkt werden. Die Vertonung und Visualisierung von normalerweise unsichtbaren Prozessen im Körper jeder der Personen kann dazu genutzt werden die soziale Kompetenz, emotionale Intelligenz und Aufmerksamkeit jeder der Personen zu stärken und zu trainieren. Zudem bietet das System 1 eine Unterstützung bei der Überwindung von sprachlichen und kulturellen Barrieren der das System 1 benutzenden Personen untereinander.

Gemäß einer Ausführungsform des erfindungsgemäßen Systems 1 kann die Rechnereinheit 3, wie in Figur 1 dargestellt, einen oder mehrere Computer umfassen, wobei einer der Computer als Relay-Server fungiert und einer der Computer als Visualisierungsrechner ausgeführt ist. Die Rechnereinheit 3 kann auch als ein Server ausgeführt sein, auf welchem mehrere virtuelle Maschinen definiert sind, welche jeweils als Relay-Server und/oder Visualisierungsrechner ausgebildet sind. Gemäß dieser Ausführungsvariante des erfindungsgemäßen Systems 1 erfassen die Wearables 5 die Parameter und berechnen Durchschnittswerte von diesen. Die Roh- und Durchschnittswerte werden in weiterer Folge an den Relay-Server übermittelt. Der Relay-Server sammelt alle Daten aller Nutzer*innen der Wearables 5. Der Relay-Server speichert diese Daten ab und stellt sie dem Visualisierungsrechner zur Verfügung. Die Daten können von dem Relay-Server auch weiteren Schnittstellen übermittelt werden. Der Relay-Server oder der Visualisierungsrechner speichert auch alle Daten in eine Datenbank. Der Visualisierungsrechner verbindet sich zum Relay-Server und empfängt alle zuvor gesammelten Daten, und errechnet aus diesen sinnvolle Werte zur Steuerung der Ausgabemittel 4 wie beispielsweise Werte für die Wiedergabe von Ton und Bild wie Lautstärken, Pitch, und/oder Geschwindigkeiten.

Im Rahmen einer Ausführungsvariante des erfindungsgemäßen Systems 1 kann eine Zuordnung der Benutzerinnen in mehreren Ebenen realisiert werden. Beispielsweise leuchtet ein Wearable 5, welches von einer Benutzerin bzw. einer Person getragen wird, in einer speziellen Farbe, und die Teile der Ausgabe z.B. der Projektion, welche die Daten dieser Person repräsentieren sind in derselben Farbe gehalten bzw. kodiert. Dies wird als Repräsentation in der Ausgabeumgebung 2 bezeichnet. Diese Kodierung kann auch über andere Wege wie Zahlen, Wörter, Formen und Bilder erfolgen. Um dem erfindungsgemäßen System 1 die Verbindung von einem Wearable 5 und den jeweiligen Daten zu anderen Teilen des Systems 1 wie dem 2D-LiDAR Tracking zu ermöglichen, z.B. um zugewiesen zu werden, gehen die Benutzerinnen an einen gekennzeichneten Ort in der Ausgabeumgebung 2 bzw. in einem Raum. Die Kennzeichnung dieses Ortes korrespondiert mit einer Kennzeichnung an dem Wearable 5 welches von der jeweiligen Person getragen wird, beispielsweise mit der Farbe.

Tritt ein Benutzer bzw. eine Benutzerin in einen der farblich kodierten und nummerierten Kreise, so wird ihr eine Tracking ID , beispielsweise vom 2D LiDAR Trackingsystem zugewiesen. Hierdurch wird eine Zuordnung des jeweiligen Wearables 5 zu den Positionen im Raum ermöglicht. Zudem wissen die Nutzer*innen hierdurch welche Repräsentation in der Ausgabeumgebung 2 ihnen zugeordnet ist. Dies kann beispielsweise mittels Projektionen von verschiedenfarbigen Kreisen in der Ausgabeumgebung 2 erfolgen, welche zusätzlich nummeriert sein können. Die Farbe und/oder die Nummer des jeweiligen Wearables 5 entspricht dann der Farbe und/oder der Nummer des Kreises.

Die Ausgabe bzw. die Projektion kann auch so gestaltet werden, dass Nutzer*innen keine Identifikationsmöglichkeit mit dezidierten grafischen oder auditiven Elementen haben. Es kann z.B. nur ein Durchschnittswert aller Nutzer*innen sicht- und hörbar gemacht werden.

Alternativ kann auch eine Verortung direkt am Wearable erfolgen, mit den hierin beschriebenen Mitteln, wodurch keine gesonderte Zuordnung notwendig ist.

## Patentansprüche

1. System (1) zur dynamischen Steuerung einer Ausgabeumgebung (2) umfassend eine Rechnereinheit (3), und zumindest ein durch die Rechnereinheit (3) steuerbares Ausgabemittel (4), wobei das System (1) dazu ausgebildet ist mittels des Ausgabemittels (4) in der Ausgabeumgebung (2) eine durch die Rechnereinheit (3) generierte Ausgabe auszugeben, **dadurch gekennzeichnet, dass**
das System (1) zumindest zwei, an jeweils einer Person anbringbare Wearables (5) umfasst, wobei die Wearables (5) mit der Rechnereinheit (3) verbunden, und dazu ausgebildet sind, zumindest einen physiologischen Parameter der das jeweilige Wearable (5) tragenden Person im Wesentlichen kontinuierlich zu erfassen und an die Rechnereinheit (3) zu übertragen, und die Rechnereinheit (3) dazu ausgebildet ist, die Ausgabe anhand aller durch die Wearables (5) erfassten physiologischen Parameter zu steuern.

2. System (1) gemäß Anspruch 1, **dadurch gekennzeichnet, dass** das System (1) im Bereich der Ausgabeumgebung (2) angeordnete, mit der Rechnereinheit (3) verbundene Erfassungsmittel (8) zur Erfassung von Umweltbedingungen in der Ausgabeumgebung (2) und/oder physiologischen Parametern von in der Ausgabeumgebung (2) befindlichen Personen umfasst, wobei die Rechnereinheit (3) dazu ausgebildet ist, die Ausgabe anhand aller durch die Wearables (5) und die Erfassungsmittel (8) erfassten physiologischen Parameter und durch die Erfassungsmittel (8) erfassten Umweltbedingungen zu steuern.

3. System (1) gemäß einem der Ansprüche 1 oder 2, **dadurch gekennzeichnet, dass** der von dem jeweiligen Wearable (5) erfasste physiologische Parameter der Person Puls, Körpertemperatur, elektrodermale Aktivität, Sauerstoffsättigung im Blut, EEG-Daten, EKG-Daten, Audiodaten, Beschleunigungsmessdaten, Lagedaten, Positionsdaten, Magnetdaten, Augenposition, Blickrichtung, und/oder Atemfrequenz der Person umfasst.

4. System (1) gemäß einem der Ansprüche 1 bis 3, **dadurch gekennzeichnet, dass** die Rechnereinheit (3) dazu ausgebildet ist, aus allen erfassten physiologischen Parametern aller jeweils mindestens ein Wearable (5) tragenden Personen ein statistisches Profil zu erstellen, und die Ausgabe anhand des statistischen Profils zu steuern.

5. System (1) gemäß einem der Ansprüche 1 bis 4, **dadurch gekennzeichnet, dass** die Wearables (5) jeweils zumindest ein mit der Rechnereinheit (3) verbundenes Ausgabemittel (4) umfassen, welches dazu ausgebildet ist, eine von der Rechnereinheit (3) generierte Ausgabe mittels des Ausgabemittels (4) des Wearables (5) direkt an die das Wearable (5) tragende Person auszugeben.

6. System (1) gemäß Anspruch 5, **dadurch gekennzeichnet, dass** das Ausgabemittel (4) des Wearables (5) ein Anzeigemittel, einen Lautsprecher, ein Heizelement, einen Ventilator und/oder ein haptisches Ausgabegerät wie eine Vibrationseinheit umfasst.

7. System (1) gemäß einem der Ansprüche 1 bis 6, **dadurch gekennzeichnet, dass** jedes Wearable (5) ein Armband, einen Brustgurt, eine Brille, ein Stirnband, eine Kopfbedeckung, einen Hut, ein Halsband und/oder eine Halskette umfasst.

8. System (1) gemäß einem der Ansprüche 1 bis 7, **dadurch gekennzeichnet, dass** das System (1) mit der Rechnereinheit (3) verbundene Positionserfassungssensoren (7) umfasst, welche dazu ausgebildet sind, eine Position zumindest einer Personen in der Ausgabeumgebung (2) zu erfassen, und die Rechnereinheit (3) dazu ausgebildet ist, die Ausgabe anhand der Position der zumindest einen Person zu steuern.

9. System (1) gemäß Anspruch 8, **dadurch gekennzeichnet, dass** die Positionserfassungssensoren (7) LiDAR-Sensoren, Kameras Netzwerktriangulationsvorrichtungen, Bluetoothtriangulationsvorrichtungen, und/oder UWB-Triangulationsvorrichtungen umfassen.

10. System (1) gemäß einem der Ansprüche 1 bis 9, **dadurch gekennzeichnet, dass** die Wearables (5) mit der Rechnereinheit (3) per WLAN, Bluetooth, Kabel, per Mobilfunknetz und/oder direkte Funkverbindung verbunden sind.

11. System (1) gemäß einem der Ansprüche 1 bis 10, **dadurch gekennzeichnet, dass** die Wearables (5) jeweils ein Eingabemittel (6) zur manuellen Eingabe eines Steuerungsbefehls und zur Übermittlung des Steuerungsbefehls an die Rechnereinheit (3) umfassen.

12. System (1) gemäß Anspruch 4, **dadurch gekennzeichnet, dass** die Erstellung des statistischen Profils die Erfassung eines Mittelwerts, eines gewichteten Mittelwerts, der Schiefe und/oder einer Standardabweichung der erfassten physiologischen Parameter der Personen umfasst.
